# EUROPEAN PATENT APPLICATION

(11) **EP 4 071 133 A1**
(43) Date of publication of application: **12.10.2022**
(21) Application number: 20910486.8
(22) Date of filing: 16.07.2020
(51) Int. Cl.: C07C 69/608, C08K 5/12, C08K 5/00, C08L 27/06

(54) **ESTER-BASED COMPOUND AND USE THEREOF**

(30) Priority: 30.12.2019 KR 20190178674
(71) Applicant: Hanwha Solutions Corporation, Jung-gu Seoul 04541 (KR)
(72) Inventor: KIM, Minjeong, Daejeon 34128 (KR); KANG, Paul, Daejeon 34128 (KR); KIM, Kidon, Daejeon 34128 (KR); KIM, Yangjung, Daejeon 34128 (KR)
(74) Representative: von Tietzen und Hennig, Nikolaus
(86) International application number: PCT/KR2020/009373
(87) International publication number: WO 2021/137375

(57) **Abstract**

Provided are a novel ester-based compound and a use thereof. A use of a novel ester-based compound for improving heat resistance and compatibility as a plasticizer is provided. When the ester-based compound according to the present invention is used as the plasticizer of a heat-resistant resin composition, a synergistic effect on improvement of physical properties such as migration resistance and heating loss is provided to contribute to improvement of the physical properties of a molded article, and due to a rapid absorption rate and a short melting time, processability of the heat-resistant resin composition may be improved.

## Description

### [Technical Field]

The present invention relates to a novel ester-based compound and a use thereof.

### [Background Art]

Polymer resins used in everyday life have been variously applied and used in each field of living and home appliances, clothing, automobiles, construction materials, packaging materials, or the like, according to each of their characteristics. In general, polymer resins selected from polyethylene (PE), polypropylene (PP), polystyrene (PS), polyurethane (PU), polyvinyl chloride (PVC), and the like are used universally. In particular, polyvinyl chloride has universal utility, since it has hard and soft properties, may be advantageously applied to various molding methods, and has excellent price competitiveness, and thus, it is applied to various fields of application ranging from household goods to industrial materials.

Polyvinyl chloride is used by adding a plasticizer thereto for implementing various physical properties, rather than used as a resin alone. The plasticizer serves to impart flexibility to the resin to improve physical properties such as processability and moldability. However, as the industry develops, the role of the plasticizer has been diversified to strengthen the properties such as volatility resistance, migration resistance, aging resistance, cold resistance, oil resistance, water resistance, and heat resistance, which are required in the field of application.

An example of an ester-based compound universally used as the plasticizer may include dioctyl terephthalate (DOTP), di-(2-ethylhexyl) phthalate (DEHP), diisononyl phthalate (DINP), di-2-propylheptyl phthalate (DPHP), diisodecyl phthalate (DIDP), or the like. However, each of these products shows inferior quality of plasticization efficiency, migration amount, or the like. In addition, there was a limitation in improving the physical properties of the product in processability with a resin, an absorption rate, volatility loss, migration loss, heat stability, and the like. In addition, considering domestic and international regulations, studies on a safe plasticizer to replace a conventional plasticizer are ongoing.

An example of a plasticizer composition which may be applied to PVC includes di(2-ethylhexyl)terephthalate and the like which are relatively inexpensive. However, it has been reported that when the composition is applied as a plasticizer, hardness and sol viscosity are high, an absorption rate of the plasticizer is relatively low, and migration and stress migration are not good. Thus, in order to make improvements, improvement of quality characteristics of the plasticizer was promoted by a method of hydrogenating terephthalate, but there is a limitation in improving quality by a basic structure of terephthalate.

Therefore, there is a need to develop a new eco-friendly plasticizer which is eco-friendly or non-phthalate-based and may sufficiently improve the physical properties of conventional products in terms of various physical properties such as processability with a resin, an absorption rate, volatility loss, migration loss, and heat stability.

### [Disclosure]

### [Technical Problem]

An object of the present invention is to provide an ester-based compound with both heat resistance and compatibility being improved and a use thereof.

Specifically, an object of the present invention is to provide an ester-based compound having a use as a plasticizer with excellent heating loss and migration resistance, an ester-based plasticizer composition including the same, and a method of preparing the same.

Specifically, another object of the present invention is to provide a vinyl chloride-based resin composition including the ester-based plasticizer composition and a molded article manufactured therefrom.

### [Technical Solution]

In one general aspect, a compound represented by the following Chemical Formula 1, that is, a novel ester-based compound is provided: 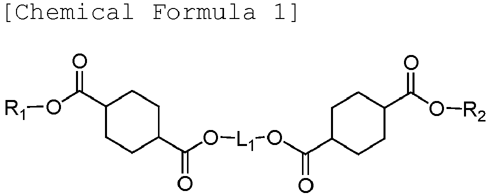 wherein
R₁ and R₂ are independently of each other C1-C10 alkyl; and
L₁ is C1-C10 alkylene, and non-adjacent -CH₂- of the alkylene may be replaced in a manner in which O atoms are not directly connected to each other.

The compound according to an exemplary embodiment of the present invention may be the compound of Chemical Formula 1 in which R₁ and R₂ are independently of each other C3-C10 alkyl, and L is C2-C6 alkylene.

The compound according to an exemplary embodiment of the present invention may be the compound of Chemical Formula 1 in which R₁ and R₂ are independently of each other C3-C10 alkyl, and L is selected from a structure represented by the following Chemical Formula A: 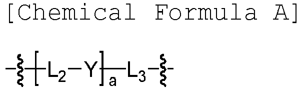 wherein
L₂ and L₃ are independently of each other ethylene or propylene;
Y is O; and
a is an integer of 1 or 2.

The compound according to an exemplary embodiment of the present invention may be the compound of Chemical Formula 1 in which R₁ and R₂ are independently of each other branched C3-C10 alkyl.

In another general aspect, an ester-based plasticizer composition includes the compound represented by Chemical Formula 1.

The ester-based plasticizer composition according to an exemplary embodiment of the present invention may include 30 wt% or more of the compound represented by Chemical Formula 1, based on a total weight.

In another general aspect, a method of preparing an ester-based plasticizer composition includes: performing a transesterification reaction of a compound represented by the following Chemical Formula 2 and a polyhydric alcohol to prepare an ester-based plasticizer composition including the compound represented by Chemical Formula 1: 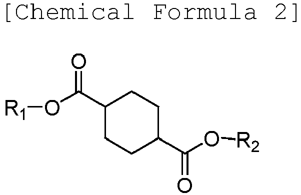 wherein
R₁ and R₂ are independently of each other C1-C10 alkyl.

In the method of preparing an ester-based plasticizer composition according to an exemplary embodiment of the present invention, the polyhydric alcohol may include an alkylene group having 2 to 10 carbon atoms.

In the method of preparing an ester-based plasticizer composition according to an exemplary embodiment of the present invention, the ester-based plasticizer composition may include 30 wt% or more of the compound represented by Chemical Formula 1, based on a total weight.

In the method of preparing an ester-based plasticizer composition according to an exemplary embodiment of the present invention, the ester-based plasticizer composition may include a compound represented by the following Chemical Formula 2 as a remainder, based on the total weight: 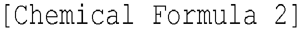 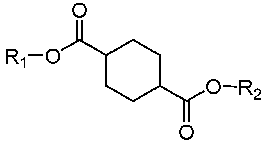 wherein
R₁ and R₂ are independently of each other C1-C10 alkyl.

In another general aspect, a vinyl chloride-based resin composition includes: the ester-based plasticizer composition described above.

The vinyl chloride-based resin composition according to an exemplary embodiment of the present invention may include 5 to 100 parts by weight of the ester-based plasticizer composition, based on 100 parts by weight of the vinyl chloride-based resin.

The vinyl chloride-based resin composition according to an exemplary embodiment of the present invention may further include one or more additives selected from a heat stabilizer, a filler, and the like.

The vinyl chloride-based resin composition according to an exemplary embodiment of the present invention may have a ratio of heating loss of 2.0% or less, the ratio of heating loss being measured with respect to the weight of an initial specimen measured at room temperature after being heated at 120°C for 72 hours.

Still another general aspect, a molded article manufactured from the vinyl chloride-based resin composition described above is provided.

### [Advantageous Effects]

According to the present invention, an ester-based compound which is useful as a plasticizer for improving physical properties such as heat resistance and compatibility required for a heat-resistant resin composition such as a vinyl chloride-based resin is provided. In addition, the compound may improve processability which is the problem of a conventional ester-based compound universally used as a plasticizer.

According to the present invention, an ester-based plasticizer which is particularly excellent in properties such as migration resistance and heating loss may be provided in a very economical manner. In addition, according to the present invention, a manufacturing process is simplified and manufacturing costs may be reduced.

When the ester-based plasticizer according to the present invention is used as the plasticizer of a heat-resistant resin composition, a synergistic effect to improve physical properties such as migration resistance and heating loss is provided, thereby increasing compatibility, that is, plasticization efficiency and contributing to the improvement of the physical properties of a molded article. In addition, the ester-based plasticizer according to the present invention has a rapid absorption rate and a short melting time to improve the processability of a resin composition, and does not cause the physical properties of the heat-resistant resin to be deteriorated.

### [Best Mode]

Hereinafter, the ester-based compound according to the present invention and the use thereof will be described in detail. Here, technical terms and scientific terms used in the present specification have the general meaning understood by those skilled in the art to which the present invention pertains unless otherwise defined, and description for the known function and configuration which may unnecessarily obscure the gist of the present invention will be omitted in the following description.

The singular form used in the present specification may be intended to also include a plural form, unless otherwise indicated in the context.

In addition, units used in the present specification without particular mention are based on weights, and as an example, a unit of % or ratio refers to a wt% or a weight ratio and wt% refers to wt% of any one component in a total composition, unless otherwise defined.

In addition, the numerical range used in the present specification includes all values within the range including the lower limit and the upper limit, increments logically derived in a form and span in a defined range, all double limited values, and all possible combinations of the upper limit and the lower limit in the numerical range defined in different forms. Unless otherwise defined in the specification of the present invention, values which may be outside a numerical range due to experimental error or rounding of a value are also included in the defined numerical range.

The term "comprise" in the present specification is an open-ended description having a meaning equivalent to the term such as "is/are provided", "contain", "have", or "is/are characterized", and does not exclude elements, materials, or processes which are not further listed.

The term "alkyl" in the present specification refers to a monovalent radical derived from an aliphatic hydrocarbon in the form of a linear or branched chain. In addition, "alkylene" refers to a divalent radical derived from an aliphatic hydrocarbon in the form of branched chain, ethylene may have a structural formula of -CH₂CH₂-, and propylene may have a structural formula of -CH₂CH₂CH₂- or-CH(CH₃)CH₂-.

The term "compound according to the present invention" or "compound of the present invention" in the present specification refers to the compound represented by the following Chemical Formula 1, that is, an ester-based compound. In addition, the ester-based compound has a use as a plasticizer, and may have an equivalent meaning to an ester-based plasticizer.

The present inventors deepened the study of a plasticizer for improving heat resistance as well as processability with a heat-resistant resin such as a vinyl chloride-based resin, and invented a novel ester-based compound, that is, an ester-based plasticizer by a combination of alcohols having different structural characteristics from each other. In addition, the ester-based compound according to the present invention includes both a substituent derived from a monohydric alcohol and a substituent derived from a polyhydric alcohol having different structural characteristics from each other. It was confirmed that the ester-based compound having the structural characteristics as such may achieve the object described above, and in particular, may implement a surprisingly improved synergistic effect on migration resistance and volatility resistance, and thus, the present invention is proposed.

The present invention is characterized by including substituents having different structural characteristics from each other, that is, both a substituent derived from a monohydric alcohol and a substituent derived from a polyhydric alcohol, and has an alicyclic dicarboxylic acid as a basic structure, and thus, may be an eco-friendly plasticizer.

The compound according to the present invention, that is, an ester-based compound effectively prevents a problem caused by its release when used as a plasticizer of a heat-resistant resin. In addition, the ester-based compound has a high molecular weight, but implements equivalent hardness properties, as compared with a universally used ester-based compound, and thus, provides an advantage which is good for compatibility, such as processability and workability, thereby having high plasticization efficiency. That is, according to the present invention, an ester-based compound which may satisfy both heat resistance and compatibility, and a use thereof may be provided. In addition, according to the present invention, an ester-based compound allowing implementation of the physical properties described above may be provided in a very economical manner.

Hereinafter, the present invention will be described in detail.

The ester-based compound according to an exemplary embodiment of the present invention may be represented by the following Chemical Formula 1: 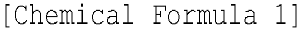 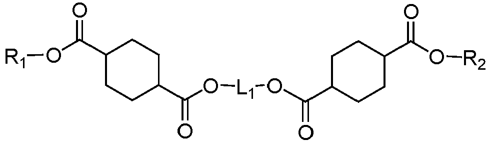 wherein
R₁ and R₂ are independently of each other C1-C10 alkyl; and
L₁ is C1-C10 alkylene, and non-adjacent -CH₂- of the alkylene may be replaced in a manner in which O atoms are not directly connected to each other.

The non-adjacent -CH₂- of the alkylene refers to alkylene except -CH₂- adjacent to an oxygen atom connected to both ends of L₁ by a direct bond.

The ester compound represented by Chemical Formula 1 is a non-phthalate-based plasticizer having the structural characteristics described above, and improves inferior quality of cyclohexane diester (DEHCH) and the like which are universally used conventional ester-based compounds. The cyclohexane diester has low viscosity at room temperature (25°C) and at a low temperature to implement excellent coating properties, but when it is used alone as the plasticizer of a vinyl chloride-based resin, it causes a problem in a migration amount.

The ester-based compound may satisfy the following structures, in terms of showing a synergistic effect on the effect as such.

As an example, in Chemical Formula 1, R₁ and R₂ may be independently of each other C3-C10 alkyl, and L may be C2-C6 alkylene.

As an example, in Chemical Formula 1, R₁ and R₂ may be independently of each other C3-C10 alkyl, and L may be selected from the structure represented by the following Chemical Formula A: 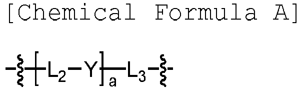 wherein
L₂ and L₃ are independently of each other ethylene or propylene;
Y is O; and
a is an integer of 1 or 2.

As an example, in Chemical Formula 1, R₁ and R₂ may be independently of each other branched C3-C10 alkyl.

As an example, in Chemical Formula 1, R₁ and R₂ may be independently of each other branched C5-C10 alkylene, and L₁ may be linear C2-C6 alkylene.

As an example, R₁ and R₂ may be independently of each other selected from methylbutyl, ethylbutyl, dimethylbutyl, methylpentyl, ethylpentyl, dimethylpentyl, methylhexyl, ethylhexyl, dimethylhextyl, and the like, and L₁ may be ethylene, propylene, butylene, pentylene, or hexylene, as linear alkylene.

As an example, R₁ and R₂ may be both 1-ethylhexyl or 2-ethylhexyl, and L₁ may be ethylene, propylene, butylene, pentylene, or hexylene, as linear alkylene.

As an example, in Chemical Formula 1, R₁ and R₂ may be independently of each other branched C5-C10 alkylene, and L₁ may be selected from the structure represented by Chemical Formula A. Here, in Chemical Formula A, L₂ and L₃ may be independently of each other ethylene or propylene, Y may be O, and a may be an integer of 1 or 2.

The ester-based compound according to an exemplary embodiment of the present invention is excellent in terms of properties such as migration resistance and heating loss, and also, improves the physical properties of a heat-resistant resin employing the ester-based compound, such as hardness, elongation, and tensile strength. In addition, the ester-based compound implements improved plasticization efficiency.

Specifically, the ester-based compound according to the present invention has a use as a plasticizer. That is, the ester-based compound according to the present invention may be an ester-based plasticizer.

In addition, the present invention provides an ester-based plasticizer composition including the ester-based compound according to the present invention and a method of preparing the same.

The ester-based plasticizer composition according to an exemplary embodiment of the present invention as an embodiment may include 30 wt% or more of the ester-based compound represented by Chemical Formula 1, based on the total weight.

The ester-based plasticizer composition according to an exemplary embodiment of the present invention may include a compound represented by the following Chemical Formula 2 as a remainder, based on the total weight. 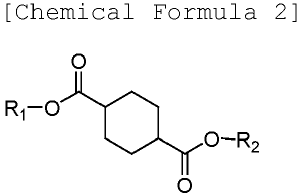 wherein
R₁ and R₂ are independently of each other C1-C10 alkyl.

The compound represented by Chemical Formula 2 may be a starting material used in the preparation of the compound represented by Chemical Formula 1, and provides an advantage of dramatically improved plasticization efficiency, migration, and the like by the combination of the compounds.

The ester-based plasticizer composition according to an exemplary embodiment provides an advantage of being excellent in heating loss according to a test method. Thus, the ester-based plasticizer according to the present invention may provide both excellent physical properties as a plasticizer and an eco-friendly plasticizer composition.

As an example, a heating loss of the ester-based plasticizer composition may be 2.0% or less.

As an example, the heating loss of the ester-based plasticizer composition may be 1.5% or less.

As an example, the heating loss of the ester-based plasticizer composition may be 0.01 to 1.4%.

The ester-based plasticizer composition according to an exemplary embodiment of the present invention may be an ether-free plasticizer composition. Here, "ether-free" means that an ether compound is substantially not included or included at 1,000 ppm or less, 100 ppm or less, or 10 ppm or less in the ester-based plasticizer.

The ester-based plasticizer composition according to an exemplary embodiment of the present invention as an embodiment may be prepared by performing a transesterification of a compound represented by the following Chemical Formula 2 and a polyhydric alcohol. 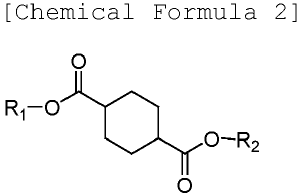 wherein
R₁ and R₂ are independently of each other C1-C10 alkyl.

The transesterification reaction refers to an interchange reaction of substituents by a reaction of the polyhydric alcohol and the compound represented by Chemical Formula 2. When the transesterification reaction is made, the alkoxide of the polyhydric alcohol attacks each carbon of two molecules of the ester groups of Chemical Formula 2 to form the compound of Chemical Formula 1. In addition, the compound of Chemical Formula 2 may remain as an unreacted part which is not reacted.

In addition, the transesterification reaction does not cause a wastewater problem as compared with the esterification reaction between acid and alcohol, and may be performed in the absence of a catalyst.

As described above, since the ester-based plasticizer composition according to the present invention has substituents derived from a combination of alcohols having different structural characteristics from each other, the ester-based plasticizer prepared therefrom may implement a surprisingly improved synergistic effect on migration resistance, volatility resistance, and the like. The synergistic effect as such is expected from the fact that the plasticizer does not leak out of the vinyl chloride resin due to an interaction such as a hydrogen bond between the ester group of Chemical Formula 1 and the ester group of Chemical Formula 2, but the synergistic effect of the ester-based plasticizer composition according to the present invention does not depend on the characteristic as such.

The ester-based plasticizer composition according to an exemplary embodiment of the present invention as an embodiment may be prepared by a hydrogen reaction of a compound of the following Chemical Formula 3 having terephthalate as a basic structure: 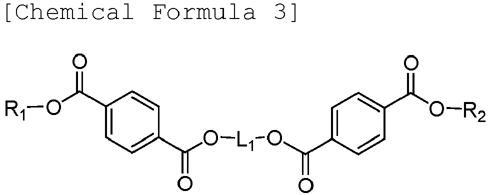 wherein
R₁ and R₂ are independently of each other C1-C10 alkyl; and
L₁ is C1-C10 alkylene, and non-adjacent -CH₂- of the alkylene may be replaced in a manner in which O atoms are not directly connected to each other.

The hydrogenation reaction may be performed by a hydrogenation reaction in the presence of a metal catalyst, and the reaction may be a kind of reduction reaction. Here, the metal catalyst may be used without limitation as long as it allows a hydrogenation reaction, and a non-limiting example thereof may include a Rh/C catalyst, a Pt catalyst, a Pd catalyst, and the like.

According to the method of preparing an ester-based plasticizer composition according to the present invention described above, the molecular weight of a plasticizer is improved, and the number of functional groups capable of interacting with a resin, that is, ester groups, is increased. In general, the aging resistant physical properties and the like of a plasticizer depends on a molecular weight, structural characteristics, and the like, and as the molecular weight of the plasticizer is higher, the aging resistant physical properties are better, but compatibility with a resin tends to be reduced. However, according to the present invention, it does not follow the trends of the conventional technologies in that both aging resistant physical properties and compatibility with a resin may be improved.

In addition, according to the present invention, an ester-based plasticizer composition of an embodiment in which at least two or more compounds are mixed is provided. This exerts an excellent synergistic effect on migration resistance and volatility resistance. Specifically, the ester-based plasticizer composition according to an exemplary embodiment of the present invention may be an embodiment in which at least one compound selected from the ester-based compound represented by Chemical Formula 1 and at least one compound selected from the compound represented by Chemical Formula 2 are mixed.

As an example, the ester-based plasticizer composition prepared by the preparation method according to the present invention may show a heating loss of 50% or less as compared with a cyclohexane diester (DEHCH) single compound. Here, the heating loss may be a ratio of heating loss measured with respect to the weight of an initial specimen measured at room temperature after being heated at 120°C for 72 hours.

As an example, the heating loss of the ester-based plasticizer composition may be 2.0% or less.

As an example, the heating loss of the ester-based plasticizer composition may be 1.5% or less.

As an example, the heating loss of the ester-based plasticizer composition may be 0.01 to 1.4%.

As an example, the ester-based plasticizer composition prepared by the preparation method according to the present invention may show a significantly low migration amount as compared with a cyclohexane diester (DEHCH) single compound. Here, the migration amount may be calculated by measuring the weight according to the following evaluation method and using the equation of (Wq2-Wq1)/Wi × 100.

In addition, the ester-based plasticizer composition prepared by the preparation method according to the present invention has improved heating loss and migration amount, and excellent tensile strength, elongation, and heat aging resistance (e.g., tensile residual rate after heating, stretch residual rate, and the like).

In the method of preparing an ester-based plasticizer composition according to an exemplary embodiment of the present invention, 0.1 to 50 parts by weight, specifically 0.5 to 30 parts by weight, and more specifically 1 to 20 parts by weight of the polyhydric alcohol may be included, based on 100 parts by weight of the compound represented by Chemical Formula 1.

When the weight ratio as such is satisfied, a plasticizer composition having a significantly reduced migration amount in the resin may be provided, which is thus preferred. In addition, the content of alcohol present as unreacted or an ether compound produced as a by-product is decreased to increase process efficiency. In addition, an ester-based plasticizer composition having high process efficiency and excellent processability and absorption rate is obtained.

In the method of preparing an ester-based plasticizer composition according to an exemplary embodiment of the present invention, the composition and the physical properties of the ester-based plasticizer composition to be desired may be controlled by the amount of the polyhydric alcohol used.

In the method of preparing an ester-based plasticizer composition according to an exemplary embodiment of the present invention, in Chemical Formula 1, R₁ and R₂ may be independently of each other branched C3-C10 alkyl, and in terms of implementing appropriate hardness and providing an advantage of mechanical properties of a resin, R₁ and R₂ may be preferably 1-ethylhexyl, 2-ethylhexyl, or a combination thereof.

In the method of preparing an ester-based plasticizer composition according to an exemplary embodiment of the present invention, the polyhydric alcohol may be a compound including an alkylene group having 2 to 10 carbon atoms. A non-limiting example thereof may be selected from glycerol, neopentyl glycol, ethylene glycol, diethylene glycol, triethylene glycol, propylene glycol, dipropylene glycol, tripropylene glycol, 1,4-butanediol, 1,3-butanediol, 2,3-butanediol, 1,3-pentanediol, 1,5-pentanediol, 1,6-hexanediol, 1,2-hexanediol, 3-methylenepentane-1,5-diol, 1,7-heptanediol, 1,8-octanediol, 1,9-nonanediol, 2-methyloctane-1,8-diol, and the like.

In the method of preparing ester-based plasticizer composition according to an exemplary embodiment of the present invention, the step may be performed in the presence of an organometallic catalyst including Sn-based or Ti-based catalyst, an acid catalyst including a sulfonic acid-based or sulfuric acid-based catalyst, or a mixed catalyst thereof. Here, the amount of the catalyst used is not limited, and may be used at a common amount of the catalyst used.

As an example, in the organometallic catalyst, the Ti-based organometallic catalyst may be selected from tetraalkyl titanate such as tetramethyl titanate, tetraethyl titanate, tetrapropyl titanate, tetraisopropyl titanate, tetrabutyl titanate, tetrampentyl titanate, tetrahexyl titanate, tetraoctyl titanate, tetranonyl titanate, tetradodecyl titanate, tetrahexadecyl titanate, tetraoctadecyl titanate, tetradecyl titanate, and tetraheptyl titanate; and tetraaryl titanate such as tetraphenyl titanate.

As an example, the acid catalyst may be selected from sulfuric acid, paratoluene sulfonic acid, methanesulfonic acid, ethanesulfonic acid, propanesulfonic acid, butanesulfonic acid, and the like.

As an example, the catalyst may be used at a common use amount, and specifically, may be used at 0.01 to 1 part by weight, based on a total of 100 parts by weight of the terephthalic acid, the monohydric alcohol, and the polyhydric alcohol, but is not limited thereto.

In the method of preparing an ester-based plasticizer composition according to an exemplary embodiment of the present invention, the step may be performed in the absence of a catalyst, of course.

In the method of preparing an ester-based plasticizer composition according to an exemplary embodiment of the present invention, the step may be performed by batch injection of the compound represented by Chemical Formula 2 and the polyhydric alcohol. In addition, the step may be performed by continuous injection of continuous injecting the mixture of the compound represented by Chemical Formula 2 and the polyhydric alcohol, of course.

In the method of preparing an ester-based plasticizer composition according to an exemplary embodiment of the present invention, the step may be performed by initiating the transesterification reaction at a reaction temperature of 80°C or higher.

As an example, the reaction temperature of the step may be 135°C or higher.

As an example, the reaction temperature of the step may be 150°C or higher.

As an example, the reaction temperature of the step may be 170°C to 270°C.

As an example, the step may be performed in multiple steps at different reaction temperatures from each other. Specifically, the step may include a first step performed at a reaction temperature of 170 to 200°C; and a second step performed under a reaction temperature of 210 to 250°C.

As an example, the step may be performed at the reaction temperature described above for 10 minutes to 24 hours, and the reaction time may be appropriately adjusted depending on the purpose, of course.

In addition, the step may be performed under an inert atmosphere. The inert atmosphere refers to an inert gas atmosphere selected from nitrogen, argon, and the like.

The method of preparing an ester-based plasticizer composition according to an exemplary embodiment of the present invention may further include a purification step including a step of recovering unreacted alcohol, reaction by-products, or both of them, or the like, after the step. The unreacted alcohol recovered by the purification as such may be reused, and thus, is advantageous in that it is continuously used in the reaction step to provide a more economical process.

The step of recovering the unreacted alcohol may be a step of removing alcohol as a reaction by-product as well as the polyhydric alcohol present in an unreacted state, and may be a distillation step using a boiling point difference. When the distillation step is used, it is preferred that a difference in boiling points of the materials to be separated is 10°C or more. In addition, the distillation may be multistage distillation or mixed distillation. The multistage distillation may be separate distillation depending on the boiling point difference of each material to be separated, and the mixed distillation may be simultaneous distillation of the materials to be separated.

In addition, the present invention provides an ester-based plasticizer composition including the ester-based compound prepared from the preparation method described above.

Specifically, the ester-based plasticizer composition according to the present invention may include a compound represented by the following Chemical Formulae 1 and 2. Specifically, the ester-based plasticizer composition may include 30 wt% or more of the ester-based compound represented by Chemical Formula 1. 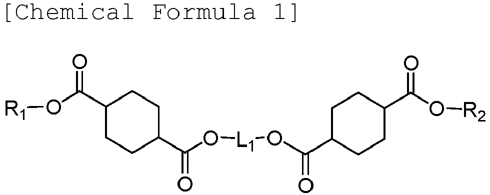 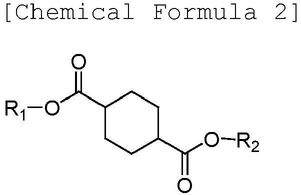 wherein
R₁ and R₂ are independently of each other C1-C10 alkyl; and
L₁ is C1-C10 alkylene, and non-adjacent -CH₂- of the alkylene may be replaced in a manner in which O atoms are not directly connected to each other.

As an example, in Chemical Formulae 1 and 2, R₁ and R₂ may be independently of each other C3-C10 alkyl, and L₁ may be C2-C6 alkylene.

As an example, in Chemical Formulae 1 and 2, R₁ and R₂ may be independently of each other C3-C10 alkyl, and L₁ may be selected from the structure represented by the following Chemical Formula A: 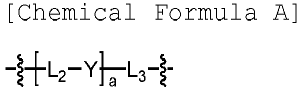 wherein
L₂ and L₃ are independently of each other ethylene or propylene;
Y is O; and
a is an integer of 1 or 2.

As an example, in Chemical Formulae 1 and 2, R₁ and R₂ may be independently of each other branched C3-C10 alkyl.

As an example, in Chemical Formulae 1 and 2, R₁ and R₂ may be independently of each other branched C5-C10 alkyl, and L₁ may be linear C2-C6 alkylene.

The ester-based plasticizer composition according to an exemplary embodiment of the present invention may be an ether-free plasticizer. Here, "ether-free" means that an ether compound is substantially not included or included at 1,000 ppm or less, 100 ppm or less, or 10 ppm or less in the ester-based plasticizer.

The ester-based plasticizer composition according to an exemplary embodiment of the present invention includes a conventional plasticizer, that is, a plasticizer such as cyclohexane diester (DEHCH), but may overcome inferior quality of plasticization efficiency, migration, and the like which may be caused therefrom. In addition, an advantage of excellence in heating loss of a specimen prepared therefrom is provided. As a result as such, the ester-based plasticizer according to the present invention has significance in providing excellent physical properties as a plasticizer and also solving the problems of a conventional plasticizer which currently causes environmental issues. That is, the present invention provides an advantage of providing an eco-friendly plasticizer composition.

In addition, the ester-based plasticizer composition according to an exemplary embodiment of the present invention may solve not only the problem of the low-molecular weight plasticizer described above, but also the problem of conventional polymer plasticizers. Specifically, though the ether-based plasticizer composition according to the present invention has a molecular weight of about 600 or more, it implements a hardness equivalent to the hardness of the low-molecular weight plasticizer described above. Thus, the ether-based plasticizer composition does not provide a disadvantage in processability with a heat-resistant resin such as a vinyl chloride-based resin, and implements high plasticization efficiency.

More specifically, according to the present invention, an ester-based plasticizer composition including 20 wt% or less of the compound represented by Chemical Formula 2 may be provided.

Most specifically, according to the present invention, an ester-based plasticizer composition including 18 wt% or less of the compound represented by Chemical Formula 2 may be provided.

The present invention provides a vinyl chloride-based resin composition including the ester-based plasticizer composition according to the present invention described above. Specifically, the vinyl chloride-based resin composition may include 5 to 100 parts by weight of the ester-based plasticizer composition, based on 100 parts by weight of the vinyl chloride-based resin.

As an example, the vinyl chloride-based resin composition may include 10 to 80 parts by weight of the ester-based plasticizer composition, based on 100 parts by weight of the vinyl chloride-based resin.

As an example, the vinyl chloride-based resin composition may include 30 to 60 parts by weight of the ester-based plasticizer composition, based on 100 parts by weight of the vinyl chloride-based resin.

The vinyl chloride-based resin composition according to an exemplary embodiment of the present invention may further include an additive selected from a heat stabilizer, a filler, and the like.

The heat stabilizer may be a composite heat stabilizer including two or more selected from Ca, Zn, Al, Mg, and the like.

As an example, 1 to 15 parts by weight of the heat stabilizer may be included, based on 100 parts by weight of the vinyl chloride-based resin. Specifically, the heat stabilizer may be included at 3 to 12 parts by weight, more specifically 5 to 10 parts by weight. When the heat stabilizer is used at the content described above, it helps to improve heat stability. In addition, since it has particularly excellent compatibility and synergistic effect with the ester-based plasticizer and the vinyl chloride-based resin according to the present invention, better effect is shown than other stabilizers. In addition, it may further include a non-metal stabilizer selected from benzophenol, triazole, acrylonitrile, and the like.

The filler may improve productivity and dry touch sensation of the vinyl chloride-based resin. The filler as such may be selected from calcium carbonate, clay, talc, diatomaceous earth, and the like.

As an example, 5 to 100 parts by weight of the filler may be included, based on 100 parts by weight of the vinyl chloride-based resin. In addition, according to the purpose, the filler may be used at a use amount of more than 100 parts by weight based on 100 parts by weight of the vinyl chloride-based resin, of course.

The vinyl chloride-based resin composition according to an exemplary embodiment of the present invention has an absorption rate and a short melting time for the vinyl chloride-based resin to increase the processability with the resin. In addition, the vinyl chloride-based resin composition according to the present invention may include a heat-resistant resin selected from an acryl resin, an ABS resin, a urethane resin, a polyester resin, and the like as well as the vinyl chloride-based resin, and also, may include various polymer resins.

The vinyl chloride-based resin composition according to the present invention implements particularly low heating loss and migration resistance, when manufactured into a wire covering material. In addition, the composition has a stretch residual rate and a tensile residual rate after excellent heating. In particular, the vinyl chloride-based resin composition according to the present invention may achieve a stretch residual rate after heating to an initial length of 80% or more, specifically 90% or more, and more specifically 95% or more, the elongation residual rate being calculated based on a length measured after heating at 120°C for 72 hours and the initial length measured at room temperature in accordance with the test method of ASTM D638. In addition, the composition may achieve a ratio of heating loss of 2.0% or less, specifically 1.5% or less, and more specifically even 1.4% or less, the ratio of heating loss being measured with respect to the initial weight measured at room temperature after being heated at 120°C for 72 hours.

In addition, the vinyl chloride-based resin composition according to the present invention may be applied to various embodiments of prescription according to the purpose. As an example, compound prescription, sheet prescription, plastisol prescription, and the like are included.

In addition, the present invention provides a molded article manufactured from the vinyl chloride-based resin composition described above. The molded article may be applied in various embodiments depending on the use and form according to the purpose, of course. Specifically, the molded article may be a wire coating material. In addition, the molded article may be flooring, wallpaper, tarpaulin, artificial leather, toy, car undercoating, or the like. In particular, according to the present invention, the migration of the plasticizer is improved so that environmental problems are not caused, and thus, a very beneficial advantage is provided.

Hereinafter, the present disclosure will be described in more detail by the following examples. However, the following examples are only a reference for describing the present invention in detail, and the present invention is not limited thereto and may be implemented in various forms. In addition, unless otherwise defined, all technical terms and scientific terms have the same meanings as those commonly understood by a person skilled in the art to which the present invention pertains. In addition, the terms used herein are only for effectively describing certain examples, and are not intended to limit the present invention.

In addition, unless otherwise stated in the present invention, the unit of temperature is all °C, and room temperature refers to 25°C.

### (Evaluation method)

### 1) Hardness (ASTM D2240):

For each specimen, the needle of a hardness tester ("A" type) was completely lowered using a hardness scale (ASKER CL-150, unit: Shore A) in accordance with the method of ASTM D2240, and then a hardness value shown at room temperature after 10 seconds was read. The hardness was tested at 5 points for each specimen, and the average value was calculated.

### 2) Tensile strength, elongation (ASTM D638):

For each specimen, a cross head speed was pulled at 200 mm/min using U.T.M., which was a test instrument, and then a tensile strength and an elongation at the point where the specimen was cut were measured. The tensile strength (kgf/cm²) was calculated by load value (kgf) / thickness (cm) × width (cm), and the elongation (%) was calculated by extension / initial strength × 100. The measurements were performed at room temperature.

In addition, the specimen was allowed to stand at 120°C for 72 hours using a gear oven, and the elongation and the tensile strength thereof were measured in the same manner. The results from the method of ASTM D638 at room temperature were divided by the results after heating to obtain a % values, which were shown as the tensile residual rate and the stretch residual rate.

### 3) Migration resistance:

Each specimen was cut into a circle having a diameter of 3 cm, and the initial weight (Wi) was measured to 4 decimal places. The specimen was inserted between two sheets of 3M oil paper (55 mm × 85 mm) of which the initial weight (Wq1) was measured and allowed to stand in an oven at 70°C for 4 days in a state of applying a load of 5 kg, and the migration amount was calculated by (Wq2-Wq1)/Wi × 100. At this time, the migration amount refers to a plasticizer outflow amount (%).

### 4) Heating loss:

For each specimen, the initial weight (Wi) was measured to 4 decimal places. The specimen was fixed in an oven at 120°C using a clamp, taken out after 72 hours, and stored in a thermostat (25°C) for 4 hours or more, the weight of the specimen (Wo) was measured, and the heating loss was calculated by (Wi-Wo)/Wi × 100.

### (Example 1)

To a 1L reactor equipped with a temperature sensor, a mechanical agitator, a condenser, a decanter, and a nitrogen injector, 500 g of cyclohexane diester (Hanwha Chemical Corporation, Eco-DEHCH) and 55.4 g of 1,4-butanediol were introduced, and the temperature was raised to 220°C with stirring under a nitrogen condition. After heating, 0.2 g of tetra-N-butyl titanate (TNBT) was introduced, the temperature was raised to 220°C, and a transesterification reaction was performed for 8 hours. When the reaction was completed, cooling to 90°C was performed, a 1 M NaOH solution was introduced, and filtration was performed using diatomaceous earth. Thereafter, unreacted alcohol, water, and impurities were removed using a rotary evaporation concentrator, and an ester-based plasticizer composition as the final product was obtained.

The obtained ester plasticizer composition was analyzed by GPC to confirm a DEHCH content (%), based on the total weight. The results are shown in the following Table 1.

In addition, the ester-based plasticizer composition prepared by the preparation method was used to manufacture a specimen. The manufacture of the specimen was performed by blending 400 g of PVC (polymerization degree: 1000) with 200 g of a plasticizer, 32 g of a composite heat stabilizer (RUP-110), and 80 g of calcium carbonate and working with a roll mill at 170°C for 3 minutes to manufacture a 1 mm sheet. Thereafter, press working was performed by preheating at 180°C for 3 minutes, heating for 9 minutes, and cooling for 3 minutes to manufacture a specimen having a thickness of 3 mm.

The specimen was used, and the results of the physical properties measured by the evaluation method are shown in Tables 1 and 2.

### (Examples 2 to 4)

An ester-based plasticizer composition was obtained in the same manner as in Example 1, except that the introduction amount of the polyhydric alcohol was changed as in the following Table 1.

The DEHCH contents (%) of the obtained ester plasticizer composition based on the total weight were confirmed by GPC analysis, and the results are shown in the following Table 1.

In addition, the specimen was manufactured in the same manner as in Example 1, and the results of the physical properties measured by the evaluation method are shown in the following Tables 1 and 2.

### (Comparative Example 1)

Commercially available cyclohexane diester (Hanwha Chemical Corporation, Eco-DEHCH) was used.

The specimen was manufactured in the same manner as in Example 1, and the results of the physical properties measured by the evaluation method are shown in the following Tables 1 and 2.

**(Table 1)**

| | Example 1 | Example 2 | Example 3 | Example 4 | Comparative Example 1 |
|---|---|---|---|---|---|
| Introduction amount of diol (DEHCH mole %) | 0.5 | 1 | 1.5 | 2 | - |
| Hardness (Shore A) | 82.5 | 84.5 | 89.2 | 93.8 | 79.3 |
| Migration amount (%) | 0.04 | 0 | 0 | 0 | 0.14 |
| Heating loss (specimen, %) | 1. 37 | 1.3 | 1. 03 | 0.74 | 2.58 |
| Tensile strength (kg/cm²) | 171 | 174 | 178 | 186 | 163 |
| Elongation (%) | 484 | 482 | 486 | 421 | 444 |
| DEHCH content (%) | 34.3 | 53.8 | 47.2 | 4.2 | 100 |

**(Table 2)**

| | Examples | | | | Comparative Examples |
|---|---|---|---|---|---|
| | 1 | 2 | 3 | 4 | 1 |
| Tensile residual rate (%) | 99.1 | 99.4 | 99.9 | 100 | 98.3 |
| Stretch residual rate (%) | 98.9 | 99.2 | 99.5 | 100 | 98.6 |

As shown in Table 1, it is recognized that when the plasticizer composition including the ester-based compound according to the present invention was employed, an excellent effect on migration resistance and heating loss was shown. Specifically, according to the present invention, it was confirmed that a migration amount of 28.6% or less (Example 1) as compared with Comparative Example 1 was implemented, and the migration amount was almost not confirmed. In addition, it was confirmed that a heating loss of 53.1% or less (Example 1) as compared with Comparative Example 1 was implemented and a significantly low heating loss of 28.6% was implemented. In addition, according to the present invention, the tensile strength and the elongation which were equivalent to or higher than cyclohexane diester (Hanwha Chemical Corporation, Eco-DEHCH) used in Comparative Example 1 were implemented, and thus, it is expected that the present invention may be usefully applied as a material to replace DEHCH.

As shown in Table 2, when the plasticizer composition including the ester-based compound according to the present invention was employed, the tensile strength and the elongation which were equivalent to or higher than cyclohexane diester (Hanwha Chemical Corporation, Eco-DEHCH) were able to be implemented, of course, and an advantage in the tensile residual rate and the stretch residual rate was also able to be provided.

Hereinabove, although the present invention has been described by specific matters, Examples, and Comparative Examples, they have been provided only for assisting in the entire understanding of the present invention. Therefore, the present invention is not limited to the above Examples. Various modifications and changes may be made by those skilled in the art to which the present invention pertains from this description.

Therefore, the spirit of the present invention should not be limited to the above-described exemplary embodiments, and the following claims as well as all modified equally or equivalently to the claims are intended to fall within the scope and spirit of the invention.

## Claims

1. A compound represented by the following Chemical Formula 1: 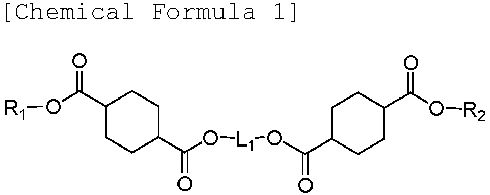 wherein
R₁ and R₂ are independently of each other C1-C10 alkyl; and
L₁ is C1-C10 alkylene, and non-adjacent -CH₂- of the alkylene may be replaced in a manner in which O atoms are not directly connected to each other.

2. The compound of claim 1, wherein in Chemical Formula 1, R₁ and R₂ are independently of each other C3-C10 alky, and L is C2-C6 alkylene.

3. The compound of claim 1, wherein in Chemical Formula 1, R₁ and R₂ are independently of each other C3-C10 alkyl, and L is selected from a structure represented by the following Chemical Formula A: 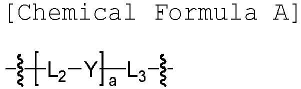 wherein
L₂ and L₃ are independently of each other ethylene or propylene;
Y is O; and
a is an integer of 1 or 2.

4. The compound of claim 1, wherein in Chemical Formula 1, R₁ and R₂ are independently of each other branched C3-C10 alkyl.

5. An ester-based plasticizer composition comprising a compound represented by the following Chemical Formula 1: 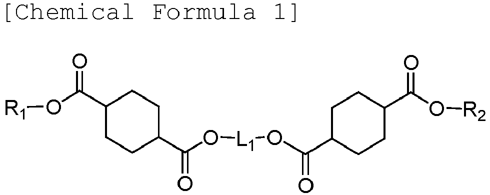 wherein
R₁ and R₂ are independently of each other C1-C10 alkyl; and
L₁ is C1-C10 alkylene, and non-adjacent -CH₂- of the alkylene may be replaced in a manner in which O atoms are not directly connected to each other.

6. The ester-based plasticizer composition of claim 5, wherein 30 wt% or more of the compound represented by Chemical Formula 1 is comprised, based on a total weight of the ester-based plasticizer composition.

7. A method of preparing an ester-based plasticizer composition, the method comprising: performing a transesterification reaction of a compound represented by the following Chemical Formula 2 and a polyhydric alcohol to prepare an ester-based plasticizer composition including a compound represented by the following Chemical Formula 1: 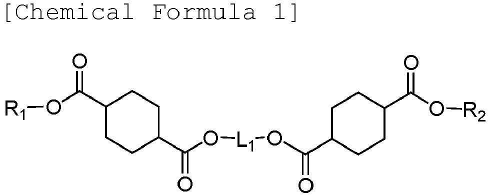 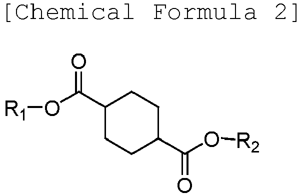 wherein
R₁ and R₂ are independently of each other C1-C10 alkyl; and
L₁ is C1-C10 alkylene, and non-adjacent -CH₂- of the alkylene may be replaced in a manner in which O atoms are not directly connected to each other.

8. The method of preparing an ester-based plasticizer composition of claim 7, wherein the polyhydric alcohol is a compound including an alkylene group having 2 to 10 carbon atoms.

9. The method of preparing an ester-based plasticizer composition of claim 7, wherein 30 wt% or more of the compound represented by Chemical Formula 1 is included, based on a total weight of the ester-based plasticizer composition.

10. The method of preparing an ester-based plasticizer composition of claim 9, wherein a compound represented by the following Chemical Formula 2 is included as a remainder, based on the total weight of the ester-based plasticizer composition: 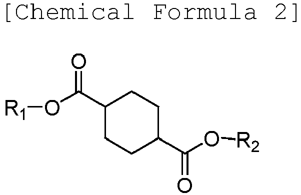 wherein
R₁ and R₂ are independently of each other C1-C10 alkyl.

11. A vinyl chloride-based resin composition comprising the ester-based plasticizer composition of claim 5 or 6.

12. The vinyl chloride-based resin composition of claim 11, wherein 5 to 100 parts by weight of the ester-based plasticizer composition is comprised, based on 100 parts by weight of the vinyl chloride-based resin.

13. The vinyl chloride-based resin composition of claim 11, further comprising: a heat stabilizer, a filler, or a combination thereof.

14. The vinyl chloride-based resin composition of claim 11, wherein the vinyl chloride-based resin composition has a ratio of heating loss of 2.0% or less, the ratio of heating loss being measured with respect to a weight of an initial specimen measured at room temperature after being heated at 120°C for 72 hours in accordance with a test method of ASTM D638.

15. A molded article manufactured from the vinyl chloride-based resin composition of claim 11.
